## Europäisches Patentamt
## European Patent Office
## Office européen des brevets

(11) Publication number: **0 073 572**
**B1**

## EUROPEAN PATENT SPECIFICATION

(45) Date of publication of patent specification: **09.04.86**

(21) Application number: **82304054.8**

(22) Date of filing: **30.07.82**

(51) Int. Cl.⁴: **A 61 G 7/04,** A 61 N 5/06,
A 47 C 7/66

(54) Sun bed.

(30) Priority: **01.08.81 GB 8123609**

(43) Date of publication of application:
**09.03.83 Bulletin 83/10**

(45) Publication of the grant of the patent:
**09.04.86 Bulletin 86/15**

(84) Designated Contracting States:
**AT BE CH DE FR IT LI NL SE**

(56) References cited:
**FR-A- 591 175**
**FR-A-2 184 495**
**GB-A-1 034 116**
**US-A-2 295 402**
**US-A-3 363 272**
**US-A-4 161 180**

(73) Proprietor: **Tolley, Derek Colin**
**Eisteddfa Farm Llannon**
**Llanelli Dyfed (GB)**

(72) Inventor: **Tolley, Derek Colin**
**Eisteddfa Farm Llannon**
**Llanelli Dyfed (GB)**

(74) Representative: **Lainé, Simon James et al**
**Wynne-Jones, Lainé & James Morgan Arcade**
**Chambers 33, St.Mary Street**
**Cardiff CF1 2AB (GB)**

Courier Press, Leamington Spa, England.

## Description

This invention relates to sun beds.

In northern countries it is sometimes possible to sunbathe outside, but for regular treatment the tendency is to rely on indoor installations using electrically powered sun lamps. These have their dangers and, of course, expense.

From US—A—2 295 402 is known a sun bed having all the features of the prior art portion of claim 1.

In FR—A—591,175 and GB—A—1 034 116 are described sun beds having air vents adjustable from the interior.

It is an aim of this invention to provide sun beds for use outside, which can take advantage of the natural rays of the sun throughout the year.

According to the present invention there is provided a sun bed comprising a base and a clear plastics dome which is closable over said base, the dome having a vent in the region of its highest point and the base having a plurality of vents around its periphery, characterised in that the base is generally flat but with a rim of inverted U-section whose outer flange is clear of the ground and whose top portion, from which the flange depends, is provided with said vents, the dome being arranged to seat around the outer periphery of that top portion and the venting being adjustable from within the bed to control convection currents which, with solar action on the dome, will draw air in through base vents while hotter air escapes through the dome vent.

For efficient weather-proofing the dome vent may be on a protruding blister.

The dome is preferably made of a clear acrylic plastics reinforced around its base by a tubular frame. The dome may be overblown so that it slightly overhangs the frame and base, giving more room and a sense of freedom for the occupant. The frame may be used to secure part of a hinge by which the dome can be attached to the base.

For a single occupant the dome and base will be elongated, with the dome larger and higher at the head end. It will be possible to have larger versions for two or more people.

The occupant may lie on a mattress on the base and this may be canted up at one end to form an inclined back and head rest.

In order to conserve heat in the colder months, a black water bed may be preferred, since this will absorb and conserve a great deal of solar energy while the bed is not in use, and keep the interior at a comfortable temperature even during periods of no sun.

For a better understanding of the invention one embodiment will now be described, by way of example, with reference to the accompanying drawing, in which:

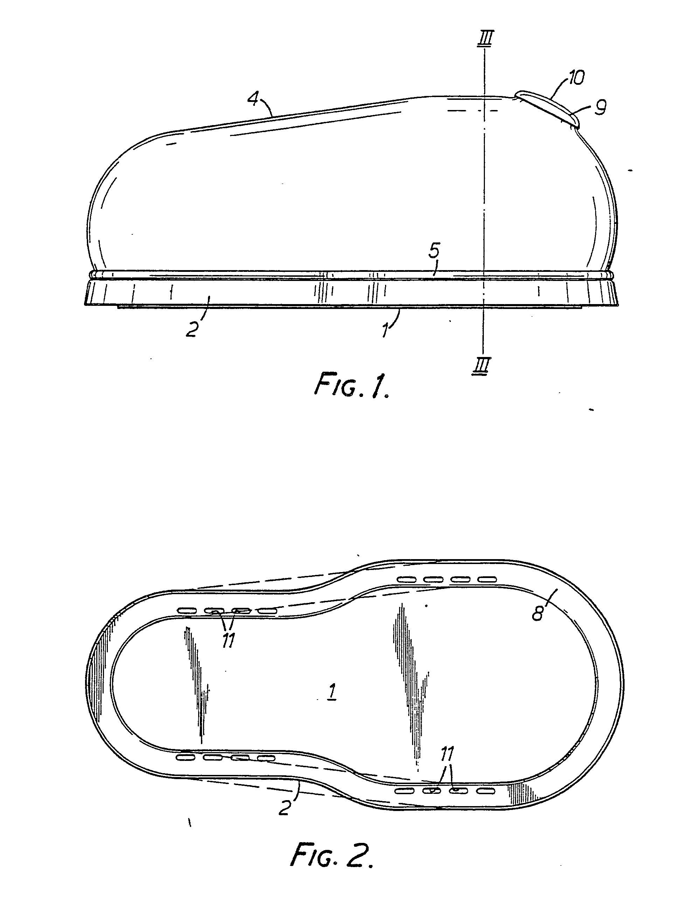

Figure 1 is a side view of a sun bed,

Figure 2 is a plan view of the base of the bed of Figure 1,

Figure 3 is a section on the line III—III of Figure 1, and

Figure 4 is a detail showing a hinge.

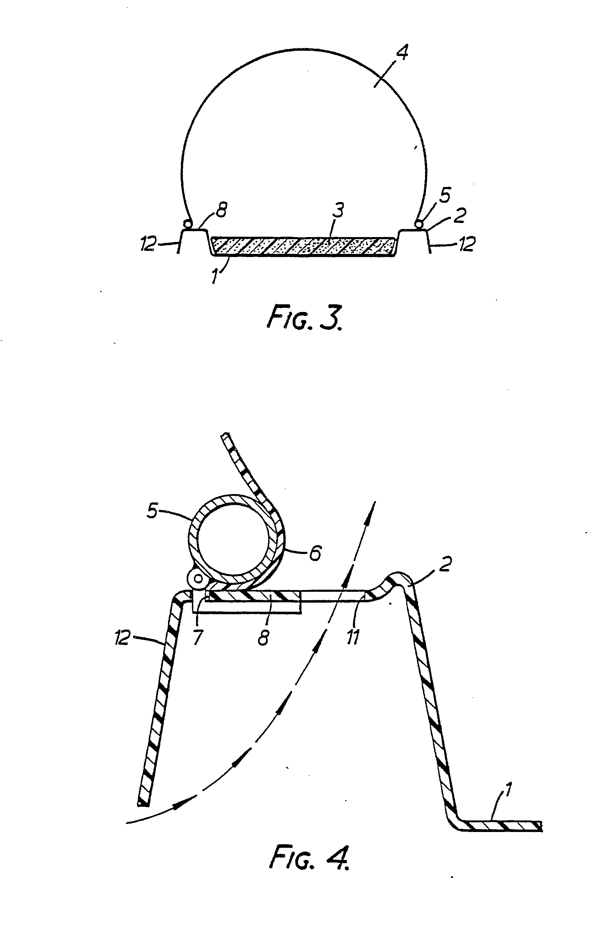

The bed has a base 1 which is generally flat but with a rim 2 of inverted U-form. In plan it is elongate, with rounded ends and with one end portion (for the head) being slightly wider than the other. Instead of these end portions having a recognisable transition with a shoulder, the sides may be straight throughout but divergent from the narrow end as indicated by the broken line. The base can be moulded in plastics or be a metal pressing, and the shallow tray formation thus provided can be used to locate a mattress 3 (Figure 3).

This base 1 is covered by a dome 4 of clear arcylic plastics material. Such material is known which has 95% permeability to the sun's radiation. The dome is reinforced around its base by a tubular frame 5, its rim portion 6 bending around, inside and under this frame. The dome is formed by a flowing process and it is distended to overhang the frame slightly, which is therefore made captive. The frame 5 follows the plan contour of the base and is used as shown in Figure 4 as a means by which the dome is hinged to the base, the other part of the hinge being entered through a slot 7 and secured to the underside of the flat top 8 of the rim 2.

The dome 4 is formed somewhat higher and wider at the headend and it is here provided, near its highest point, with a blister 9 which forms a base for a vent. The blister has a number of apertures and a clear vent cap 10 in the same plastics material shields it on the outside. However the cap 10 can be moved by the occupant from inside the dome to control the amount of closure of those apertures.

The flat top 8 of the rim 2 is formed with series of vents 11 which remain open to the inside of the dome when the latter is closed onto the outer edge portion of that top 8. The outer flange 12 of the rim 2 does not extend right down to the plane of the main portion of the base and so will be just clear of the ground. Thus air can enter under that flange into the inverted U-channel and thence via the peripheral vents 11 into the dome, as indicated by the arrowed line in Figure 4.

In use, the action of the sun will rapidly heat up the air inside the dome, and the occupant can open the vent cap 10 to allow it to escape by convection. This draws in further air via the vents 11 and a continual flow is maintained. The rate of this can be governed by the occupant to maintain a comfortable temperature. The occupant has only the highly permeable clear plastics material between him or herself and the sun, and any feeling of claustrophobia is unlikely.

## Claims

1. A sun bed comprising a base (1) and a clear plastics dome (4) which is closable over said base, the dome having a vent (9) in the region of its highest point and the base having a plurality of vents (11) around its periphery, characterised in that the base (1) is generally flat but with a rim (2) of inverted U-section whose outer flange (12) is clear of the ground and whose top portion (8), from which the flange depends, is provided with said vents (11), the dome (4) being arranged to seat around the outer periphery of that top portion (8) and the venting being adjustable from within the bed to control convention currents which, with solar action on the dome (4), will draw air in through base vents (11) while hotter air escapes through the dome vent (9).

2. A sun bed as claimed in claim 1, wherein the dome vent is on a protruding blister (9).

3. A sun bed as claimed in claim 1 or 2, wherein the dome (4) is over blown, with a reinforcing frame (5) around its rim.

4. A sun bed as claimed in claim 3, wherein the frame is used to hinge the dome to the base.

5. A sun bed as claimed in any one of the preceding claims, wherein the dome (4) and base (1) are elongated and wherein the dome (4) is larger and/or higher at one end than the other.

6. A sun bed as claimed in any one of the preceding claims, further comprising a mattress (3) on the base.

7. A sun bed as claimed in claim 6, wherein the mattress (3) is canted up at one end to form an inclined back and head rest.

8. A sun bed as claimed in claim 6 or claim 7, wherein the bed is a black water bed.

## Patentansprüche

1. Sonnenbett mit einer Basis (1) und einer klaren Plastikkuppel (4), die über die Basis geschlossen werden kann und eine Entlüftung (9) im Bereich ihrer höchsten Stelle aufweist, während die Basis entlang seines Umfanges eine Mehrzahl von Belüftungsöffnungen (11) aufweist, dadurch gekennzeichnet, daß die Basis (1) im allgemeinen flach ist, jedoch eine Rand (2) mit einem umgekehrten U-förmigen Querschnitt aufweist, dessen äußerer Flansch (12) in einem Abstand vom Boden ist und dessen Oberteil (8), von dem sich der Flansch nach unten erstreckt, mit den genannten Belüftungsöffnungen (11) versehen ist, daß die Kuppel (4) um den äußeren Umfang des Oberteils (8) herum aufliegt und daß die Lüftung von innerhalb des Bettes einstellbar ist, um die Konvektionsströmungen zu steuern, die durch die Sonneneinwirkung auf die Kuppel (4) Luft durch die Basisöffnungen (11) hereinziehen, während heißere Luft durch die Kuppelentlüftung (9) entweicht.

2. Sonnenbett nach Anspruch 1, dadurch gekennzeichnet, daß sich die Kuppelentlüftung auf einer vorstehenden Blase (9) befindet.

3. Sonnenbett nach Anspruch 1 oder 2, dadurch gekennzeichnet, daß sich die Kuppel (4) über die Basis hinauserstreckt und antlang ihrer Kante einen Verstärkungsrahmen (5) aufweist.

4. Sonnenbett nach Anspruch 3, dadurch gekennzeichnet, daß der Rahmen zur scharnierartigen Verbindung der Kuppel mit der Basis dient.

5. Sonnenbett nach einem der vorhergehenden Ansprüche, dadurch gekennzeichnet, daß die Kuppel (4) und die Basis (1) länglich sind und die Kuppel (4) an einem Ende breiter und/oder höher ist als am anderen Ende.

6. Sonnenbett nach einem der vorhergehenden Ansprüche, gekennzeichnet durch eine Matratze (3) auf der Basis.

7. Sonnenbett nach Anspruch 6, dadurch gekennzeichnet, daß die Matratze (3) an einem Ende hochgestellt ist, um eine schräge Rücken- und Kopfstütze zu bilden.

8. Sonnenbett nach Anspruch 6 oder 7, dadurch gekennzeichnet, daß das Bett ein schwarzes Wasserbett ist.

## Revendications

1. Lit pour bains de soleil comprenant une base (1) et un dome (4) transparent en matière plastique apte à être refermé au-dessus de ladite base, le dome comportant un orifice (9) dans la zone de son point culminant, et la base comportant plusieurs orifices (11) autour de sa périphérie, caractérisé en ce que la base (1) est de forme générale plate mais comporte un bourrelet (2) ayant une section en forme de "U" inversé dont le rebord (12) est espacé par rapport au sol et dont la partie supérieure (8), depuis laquelle descend le rebord, est pourvue desdits orifices (11), le dome (4) étant arrangé de manière à venir en appui autour de la périphérie extérieure de cette partie supérieure (8), et l'ouverture des orifices étant règlable depuis l'intérieur du lit pour commander les courants de convection qui, sous l'action du soleil sur le dome (4), aspirent de l'air vers l'intérieur par les orifices (11) de la base tandis que de l'air plus chaud s'échappe par l'orifice (9) du dome.

2. Lit pour bains de soleil selon la revendication 1, dans lequel l'orifice du dome est ménagé dans une bulle (9) en saillie.

3. Lit pour bains de soleil selon la revendication 1 ou 2, dans lequel le dome (4) est boursoufflé, avec un cadre de renforcement (5) autour de son bourrelet.

4. Lit pour bains de soleil selon la revendication 3, dans lequel le cadre est utilisé pour articuler le dome à la base.

5. Lit pour bains de soleil selon l'une quelconque des revendications précédentes, dans lequel le dome (4) et la base (1) sont de forme allongée, et dans lequel le dome (4) présente à une extrémité une dimension et/ou une hauteur plus grandes qu'à l'autre.

6. Lit pour bains de soleil selon l'une quelconque des revendications précédentes, comprenant en outre un matelas (3) placé sur la base.

7. Lit pour bains de soleil selon la revendication 6, dans lequel le matelas (3) est relevé à une extrémité pour former un support de dos et de tête incliné.

8. Lit pour bains de soleil selon la revendication 6 ou 7, dans lequel le lit est un lit à eau, de couleur noire.

0 073 572

FIG. 1.

FIG. 2.

FIG. 3.

FIG. 4.